Europäisches Patentamt

(19) European Patent Office

Office européen des brevets

(11) Numéro de publication : **0 032 145**
**B1**

(12) **FASCICULE DE BREVET EUROPÉEN**

(45) Date de publication du fascicule du brevet :
20.02.85

(51) Int. Cl.⁴ : **C 12 N   5/00, A 61 K 35/44,**
**G 01 N 33/48, A 61 K 35/36,**
**A 61 K   7/48**

(21) Numéro de dépôt : 80901274.3

(22) Date de dépôt : 10.07.80

(86) Numéro de dépôt international :
PCT/FR 80/00118

(87) Numéro de publication internationale :
WO/8100260 (05.02.81 Gazette 81/04)

(54) PROCEDE POUR STIMULER LA CROISSANCE DES CELLULES D'EPIDERME HUMAIN, APPLICATIONS DUDIT PROCEDE ET PRODUITS OBTENUS DE CETTE MANIERE.

(30) Priorité : 13.07.79 FR 7918282

(43) Date de publication de la demande :
22.07.81 Bulletin 81/29

(45) Mention de la délivrance du brevet :
20.02.85 Bulletin 85/08

(84) Etats contractants désignés :
AT CH DE FR GB LI LU NL SE

(56) Documents cités :
FR-A- 2 134 088
Experimental Cell Research, vol. 117, no. 2, publ. in 1978, C. Arruti et al. "Morphological Changes and Growth Stimulation of Bovine Epithelial Lens Cells by a Retinal Extract In Vitro", see pages 283 to 292
Nature, vol. 265, no. 5593, publ. on 3. February 1977, J.G. Rheinwald et al., "Epidermal Growth Factor and the Multiplication of Cultured Human Epidermal Keratinocytes", see pages 421 to 424
Chemical Abstracts, vol. 55, no. 5, publ. on 6 March 1961, (Columbus, Ohio, USA) abstract no 4892c
Chemical Abstracts, vol. 77, no. 11, publ. on 11 September 1972, (Columbus, Ohio,USA), M.A. Karasek, "Dermal Factors Affecting Epidermal Cell In Vitro", see page 305, abstract no. 73097m

(73) Titulaire : INSTITUT NATIONAL DE LA SANTE ET DE LA RECHERCHE MEDICALE (INSERM)
101, rue de Tolbiac
F-75654 Paris Cedex 13 (FR)

(72) Inventeur : GUEDON, Isabelle, née SAGLIER
23, Rue des Blancs Manteaux
F-75004 Paris (FR)
Inventeur : BARRITAULT, Denis
4, rue Française
F-75004 Paris (FR)
Inventeur : COURTOIS, Yves
11 Les Quinconces
F-91190 Gif sur Yvette (FR)
Inventeur : ARRUTI, Cristina
5, rue Cronstadt
F-75015 Paris (FR)
Inventeur : PRUNIERAS, Michel
72, Avenue Mozart
F-75016 Paris (FR)

(74) Mandataire : Phélip, Bruno et al
c/o Cabinet Harlé & Phélip 21, rue de la Rochefoucauld
F-75009 Paris (FR)

## Description

L'invention concerne des procédés et produits applicables à la peau humaine et plus particulièrement à l'épiderme humain adulte. Elle a notamment pour objet un procédé pour stimuler la croissance in vitro des cellules d'épiderme humain, en particulier adulte, et des produits cosmétiques, pharmaceutiques et de diagnostic faisant application dudit procédé.

La peau est le principal tissu qui se trouve exposé aux effets mutagènes, et, partant, potentiellement cancérigènes, des agents chimiques et physiques de l'environnement. Ceci est particulièrement vrai en ce qui concerne les radiations de toute nature. La peau est composée de tissus d'origine mésenchymateuse (le derme et les vaisseaux sanguins) et de tissus d'origine ectodermique (l'épiderme). Or, si l'on sait extraire du derme les cellules mésenchymateuses (fibroblastes) pour les cultiver et étudier les effets mutagènes et carcinogènes des facteurs chimiques et physiques de l'environnement, on éprouve des difficultés à faire de même à partir de l'épiderme. L'une de ces difficultés n'avait pas encore été surmontée. Il s'agit de l'application aux cellules épidermiques adultes en culture des procédés d'étude cytogénétique, lesquels impliquent la préparation et l'analyse des chromosomes d'une part et la préparation et la numération des échanges de chromatides sœurs (SCE) d'autre part. De fait, si quelques rares publications font état d'études chromosomiques succinctes, il n'y a pas d'étude chromosomique approfondie, en particulier dans le domaine du diagnostic des maladies humaines héréditaires touchant l'épiderme, et, à la connaissance du demandeur, il n'existe pas de référence bibliographique sur les SCE.

Une série de travaux récents permet de penser que l'augmentation des SCE est un indicateur, à la fois sensible et fidèle, de l'action mutagène d'un produit chimique ou d'une radiation. L'application des techniques de révélation des SCE aux cellules épidermiques humaines adultes est donc d'une importance certaine.

L'invention a notamment pour objet un procédé applicable à la préparation et à l'analyse des chromosomes ainsi qu'à la préparation et à la numération des SCE dans les cellules épidermiques humaines adultes en culture. Grâce à l'invention, on peut en effet induire une multiplication mitotique de ces cellules sur un substrat de verre habituellement hostile.

L'invention permet également d'apporter une solution à tous les problèmes concernant la croissance et la culture in vitro de cellules épidermiques, en particulier chez l'homme et spécialement chez l'adulte. Ainsi, outre l'application cytogénétique précédemment mentionnée, l'invention peut-elle être mise à profit dans le traitement des brûlés, dans le domaine de la cosmétique et de la pharmacologie cutanée ainsi que pour la culture de longue durée des cellules épidermiques.

La présente invention met à profit les travaux antérieurs rapportés par ARRUTI C. et COURTOIS Y. Exptl. Cell. Res. *117*, 283-292 (1978). Ces auteurs ont en effet constaté qu'un extrait rétinien, désigné par l'abréviation RE, était un facteur de croissance des cellules de l'épithélium du cristallin en culture.

L'homme de l'art pourra se reporter, si besoin est, à cet article pour y trouver les indications nécessaires concernant cet extrait RE, ainsi que les propriétés qui ont été observées. Les auteurs ont utilisé un extrait total rétinien obtenu par extraction à l'aide d'une solution saline aqueuse, en l'espèce une solution Dulbecco, en tampon phosphate à pH 7,2, par exemple du type mis sur le marché par la Société FLOW Laboratories. Les rétines de bovins sont mises en contact intime avec une telle solution. Après un certain nombre de traitements physiques de centrifugation et de filtration, on isole l'extrait rétinien RE. Toutefois, les observations faites dans cet article sont limitées à la croissance des cellules de l'épithélium du cristallin.

Certains documents de la technique antérieure font état de travaux sur des extraits provenant de tissus oculaires animaux.

Ainsi, le brevet FR 71 13 756 (publication 2 134 088) décrit un procédé pour l'obtention d'un extrait total de globe oculaire prélevé sur des bovins. La séquence d'étapes implique l'action du merthiolate, d'un alcool et de « Celite ».

L'extrait alcoolique ainsi obtenu trouve application dans le domaine ophtalmologique. Il ne s'agit donc pas d'un extrait salin aqueux et il n'est pas prévu de l'utiliser pour la croissance in vitro de cellules d'épiderme.

L'article de B. Skasavina et al. (Columbus, Ohio, USA et SU-A-130 159) du 15 Juillet 1960 cité dans Chemical Abstracts Vol. 55 n° 5 (6 mars 1961) N° 4892 c décrit la préparation d'un produit à base d'acide hyaluronique par extraction à partir d'humeur vitrée d'yeux d'animaux ainsi que son application au traitement des plaies ou blessures infectées. Le produit est un extrait chloroformique.

L'article de M. A. Karasek dans J. Invest. Dermatol. 1972 59 (1) 99-101 cité dans Chemical Abstracts Vol. 77 n° 11 (11 Septembre 1972) n° 73 097 m p. 305 traite de la croissance in vitro des cellules de l'épiderme, en présence d'un certain nombre de facteurs. Il est constaté que l'acide hyaluronique n'exerce pas d'effet stimulant sur les cellules épithéliales. Cet article montre également que l'effet d'un facteur déterminé sur la croissance des cellules de l'épiderme est impossible à prévoir à l'avance.

Il apparaît donc que la technique antérieure n'enseigne pas un facteur consistant en un extrait aqueux de tissu oculaire et efficace pour la croissance in vitro de cellules d'épiderme, en particulier des cellules d'épiderme adulte, spécialement humaines.

Selon la présente invention, on peut stimuler la croissance en culture in vitro des cellules épidermiques, indépendamment de l'âge et de l'espèce du donneur, en utilisant le facteur de croissance RE. Selon l'invention également, le facteur de croissance RE est efficace sur les cellules épidermiques adultes, et en particulier humaines.

Sous sa forme la plus générale, l'invention concerne un procédé pour stimuler la croissance in vitro des cellules d'épiderme, en particulier adulte, caractérisé en ce qu'on met en contact les cellules de l'épiderme avec un extrait salin aqueux de tissu oculaire (ou extrait RE).

Pour l'obtention de l'agent actif RE, on peut faire appel à la technique décrite dans l'article de C. ARRUTI et Y. COURTOIS mentionné précédemment. Le matériau de départ, à savoir les rétines d'yeux de bovins, est en effet aisément accessible en abondance.

Par l'expression « extrait de tissu oculaire », on désigne un produit qui peut être extrait de divers tissus de l'œil, tels que la choroïde, l'iris et l'humeur vitrée. Cette définition ne comprend pas le cristallin et l'humeur aqueuse, tissus qui ne contiennent pas de RE.

En vue de l'extraction, on utilise une solution saline aqueuse quelconque, tamponnée à un pH voisin de 7,2 et capable de fournir un extrait aqueux contenant le RE.

On a constaté avec surprise qu'en utilisant le facteur RE, selon l'invention, dans des cultures in vitro de cellules d'épidermes humains adultes, il devenait possible d'apporter une solution à des problèmes qui n'avaient pas été résolus par la technique antérieure. Ainsi, l'invention permet l'analyse chromosomique et l'évaluation des SCE dans des cultures in vitro de kératinocytes humains adultes, grâce à l'activation de l'activité mitotique des kératinocytes qui résulte de l'utilisation du RE.

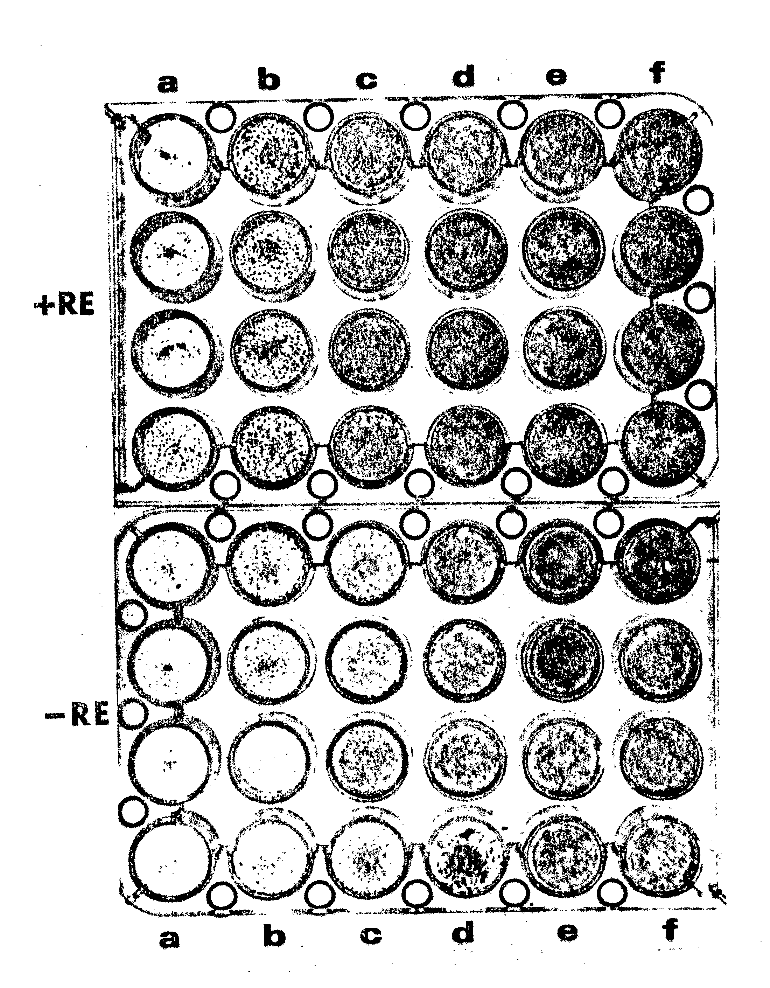

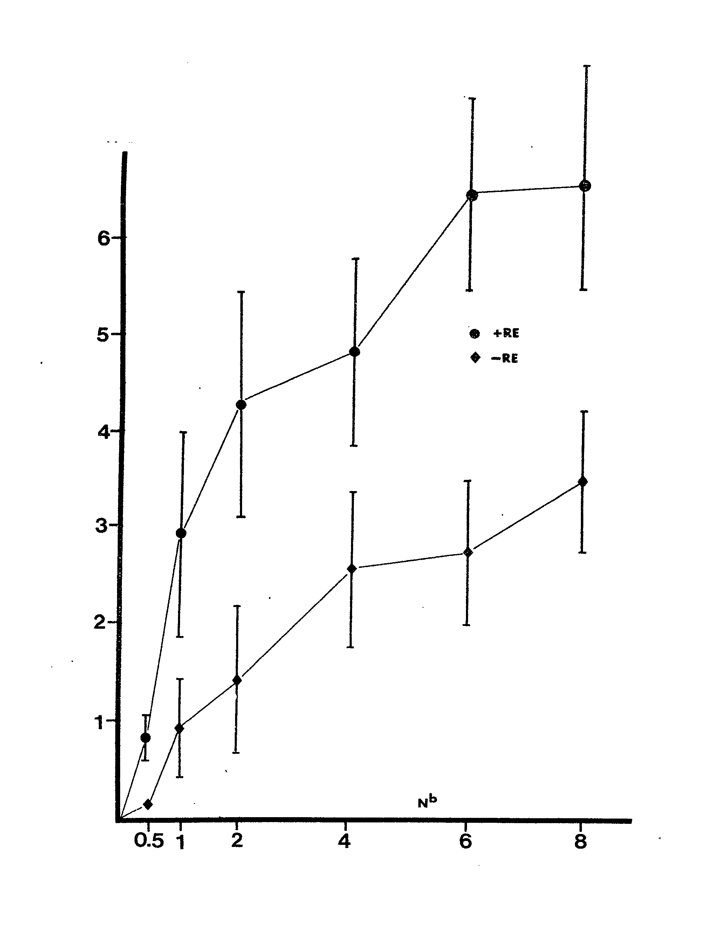

L'effet activateur du RE sur la croissance in vitro des cellules épidermiques sera illustré en référence aux figures 1 et 2 ci-après. On constate une augmentation de la surface couverte pendant un nombre de jours donné par le même nombre de cellules ensemencées. La densité cellulaire par unité de surface, révélée par coloration, est plus forte pour les cellules traitées. Cette coloration plus forte n'est pas due à une croissance en hauteur des cellules, mais bien à augmentation de l'activité mitotique.

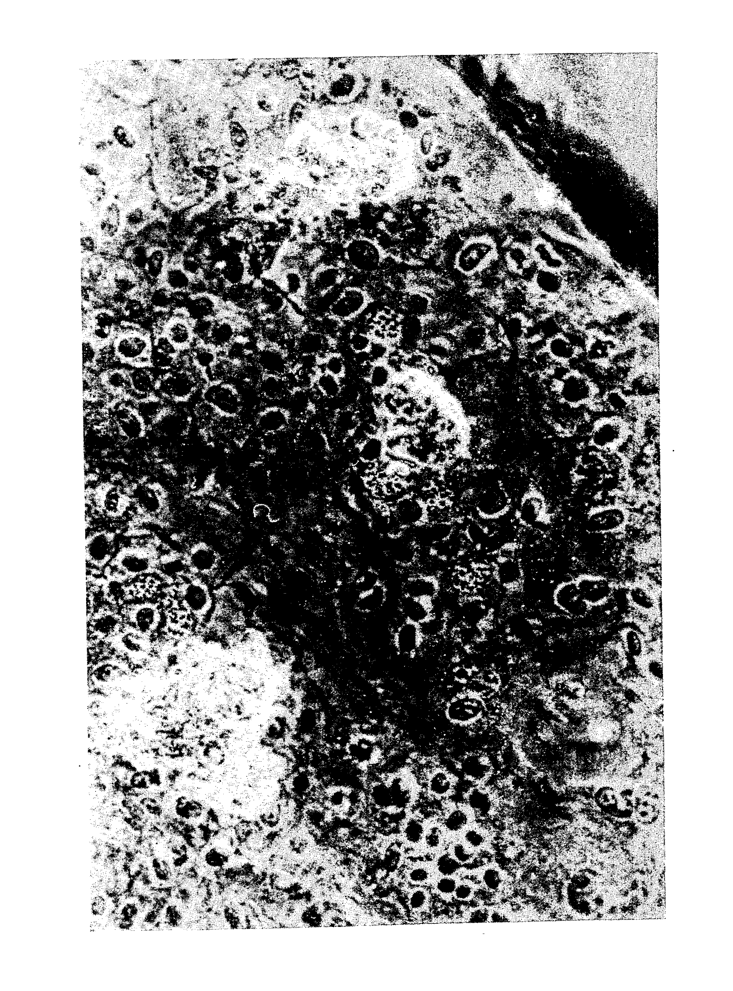

On a déjà indiqué précédemment que la préparation de chromosomes métaphasiques de cellules épidermiques humaines adultes était impossible en l'absence d'activateur de la croissance cellulaire, voir par exemple GREEN H. Cell *15*, 801-811 (1978). Des cellules sur lamelles de verre en milieu de culture ordinaire (TCM) en présence de RE permettent d'obtenir un nombre de mitoses répondant aux besoins de l'invention. Selon les constatations de l'art antérieur, les cellules primaires de kératinocytes ont tendance à s'empiler et à kératiniser. Ainsi, quelques métaphases sont situées sous une ou plusieurs couches de cellules, ce qui empêche la dispersion des chromosomes. Une partie des mitoses « spontanées » est donc perdue et le nombre restant est trop faible. En revanche, l'addition de RE induit une forte augmentation de l'activité mitotique (voir figure 3 ci-après) qui, en combinaison avec un traitement de 15 heures par le sel de colchicine, disponible sous la dénomination « Colcemid » (Gibco), permet la préparation de métaphases bien étalées en nombre suffisant. En moyenne, 20 métaphases analysables par lamelle peuvent être obtenues en routine.

L'action du RE sur les cellules épidermiques adultes diffère de celle du produit EGF (Epidermal Growth Factor) voir COHEN S. et ELLIOTT G. A., J. Invest. Dermatol., *40*, 1-5 (1963), et COHEN S. dans Hormones in Development. Hamburgh M. et Burrington E. J. ed. Meredith Corp. N.Y. 753-766 (1971) qui est essentiellement actif sur cellules fœtales ou de nouveau-né. De plus, ces deux agents mitogènes diffèrent complètement par leur action sur d'autres cellules cibles.

On a également constaté que le RE n'était pas mutagène, ce qui est une caractéristique très importante pour les cellules épidermiques adultes. Cette propriété peut d'ailleurs être mise à profit pour la détection de l'action d'agents mutagènes. Des essais ont montré que l'augmentation de la croissance de la culture cellulaire était bien due à une réelle multiplication des kératinocytes, et non à celle d'éventuels fibroblastes qui auraient pu être détachés du derme pendant la trypsinisation. Ainsi, l'examen microscopique en contraste de phase révèle que les cultures sont composées d'un seul type cellulaire, d'allure épithéliale (voir figure 3 ci-après). En outre, la réaction à la leucine-aminopeptidase (LAP) marqueur enzymatique des cellules d'origine mésenchymateuse in vivo (NACHLAS M.M., CRAWFORD D.T. et SELIGMAN A.M. J. Histochem. Cytochem. 1957, *5*, p. 264-278) et in vitro (FRITSCH P., DIEM E. Arch. Derm. Forsch *243* 364-372 (1972), REGNIER M., DELESCLUSE C. et PRUNIERAS M. Acta Dermatovener *53* 241-247 (1973) a été utilisée pour détecter les fibroblastes dermiques et s'est montrée complètement négative.

L'effet potentialisateur de croissance in vitro du RE sur les cellules épidermiques humaines adultes peut recevoir de nombreuses applications.

Dans une application donnée à titre d'exemple, l'invention permet l'analyse chromosomique et l'évaluation des SCE dans des cultures in vitro de kératinocytes humains adultes. On peut en effet préparer des chromosomes métaphasiques bien dispersés, en nombre suffisant pour l'analyse caryotypique et également décompter les SCE dans ce type cellulaire.

Les méthodes cytogénétiques, et en particulier les techniques d'évaluation des échanges de chromatides sœurs (SCE), ont été récemment développées (voir PERRY P. et WOLFF S. Nature 251, 156-158 (1974)). Cette dernière technique est d'un intérêt particulier en pharmacologie cutanée car le nombre d'échanges est directement lié à la mutagenèse (PERRY P. et EVANS H.J. Nature *258* 121-125 (1975) et CARRANO A.V., THOMPSON L.H., LINDL P.A. et MINKLER J.L., Nature *271*, 551-553 (1978)). De ce fait, la numération des SCE est précieuse pour estimer le potentiel carcinogène d'un produit cosmétique, d'une thérapeutique ou, plus généralement, d'une exposition volontaire ou accidentelle aux facteurs environne-

3

mentaux chimiques ou physiques (radiations de toute nature). Dans la littérature, il y a très peu d'informations concernant la cytogénétique des cellules épidermiques et il n'y a pas du tout de travaux sur les SCE. Cette pauvreté est sans doute due à trois difficultés majeures.

La première réside dans l'étalement des chromosomes. La trypsine est habituellement utilisée pour récolter les cellules en mitose et disperser les chromosomes. Cependant, pour récolter des kératinocytes adultes en culture, il faut utiliser de l'EDTA en plus de la trypsine (RHEINWALD J.G. et GREEN H., Cell. *6* 331-344 (1975)) et l'EDTA interfère avec le gonflement des cellules en solution hypotonique, qui constitue un impératif de base à un bon étalement chromosomique. Les chromosomes peuvent être dispersés de façon satisfaisante en traitant directement la culture in situ, mais on se heurte alors à une deuxième difficulté, car la meilleure surface pour l'étalement des chromosomes est le verre, et le verre est un substrat mauvais pour l'attachement des kératinocytes (LIU S.C. et KARASEK M.J. Invest. Dermatol. *71*, 157-162 (1978)).

La troisième difficulté est que, pour obtenir des SCE, deux cycles de replication consécutifs des mêmes cellules sont nécessaires pour la visualisation du marquage différentiel des chromatides avec la technique de substitution par la bromo deoxyuridine (BUdR) (WOLFF S. et PERRY P. Chromosoma *48*, 341-353 (1974)-KATO H. Nature *251*, 70-72 (1974)). La culture doit donc avoir une grande activité mitotique, ce qui n'est pas le cas des kératinocytes humains adultes cultivés sur substrat de verre.

Il est très important, pour la préparation des SCE, qu'un nombre appréciable de cellules se divisent de manière synchrone. Comme il a déjà été précisé plus haut, deux cycles cellulaires sont nécessaires à une même cellule pour avoir l'un de ses brins d'ADN bisubstitué par le BUdR, préliminaire indispensable à la visualisation des SCE. De plus, ces deux cycles doivent avoir lieu dans un intervalle de temps raisonnable (50-70 heures), pour les besoins pratiques.

Les cellules épidermiques en culture primaire ne sont pas synchronisées et les cellules qui se repliquent à un moment donné n'appartiennent pas nécessairement au même groupe de cellules en replication. Cela signifie que, seule une partie des cellules en replication pourra entreprendre un deuxième cycle de replication pendant la relativement courte période de temps allouée. Le grand intérêt du RE est d'induire une synchronisation suffisante pour permettre à environ 50 % des métaphases d'être bisubstituées en environ 60 heures. Les SCE peuvent alors être comptées sur environ 10 métaphases par lamelle.

Selon un mode de réalisation, on effectue une mise en culture normale préalable des cellules épidermiques en l'absence de facteur de croissance. Dans une période comprise entre 5 et 10 jours, par exemple au bout de 8 jours, on introduit une première quantité efficace de RE. Le lendemain, ou 48 heures après, on effectue une autre application de RE en vue d'obtenir une seconde replication des cellules.

Toutefois, ce mode de réalisation n'est donné qu'à titre d'exemple. En variante, on peut introduire le RE au départ, mais il faut veiller à ce que les développements de cellules soient synchronisés. Il faut donc, si l'on adopte cette variante de réalisation, introduire le RE dès l'origine dans le système de culture cellulaire, et arrêter ensuite la culture pour permettre une synchronisation des cellules.

L'invention concerne donc des produits de diagnostic permettant d'étudier les chromosomes de la cellule de l'épiderme adulte humain. De tels produits et leurs procédés de mise en œuvre peuvent permettre la détection des anomalies chromosomiques naturelles ou induites. Des facteurs inducteurs sont notamment des irradiations, par exemple sous l'effet des rayons U.V., ou résultant de phénomènes accidentels, ainsi que tous rayonnements, tels que les rayons gamma, les rayons X, les neutrons, etc... Parmi les maladies susceptibles d'être ainsi décelées, on peut citer des maladies génétiques, le psoriasis et tous les types de cancers de la peau. Les cellules kératinocytes adultes sont en effet reconnues comme cellules cibles de la cancérogenèse de la peau. L'avantage de l'invention est de permettre une étude directe des comportements cellulaires épidermiques humains et non pas d'effectuer des études in vivo, comme c'était le cas dans la technique connue, sur des échantillons partiels cellulaires, par exemple des fibroblastes.

L'invention peut être appliquée à titre préventif pour étudier le comportement des peaux dans certains environnements, par exemple pour sélectionner le personnel devant être amené à travailler en environnement nucléaire.

Dans de telles applications, l'invention met à profit le fait que le RE n'induit pas de mutagenèse. Ainsi, lorsqu'on compte les échanges de chromatides, on constate que le taux d'échange est inférieur à 3 par micose, ce qui correspond à un chiffre très faible que l'on dénomme habituellement « échanges chromatides spontanés ».

On constate également que l'extrait RE, utilisé selon l'invention, est exempt de toxicité. Lorsqu'on double la dose de RE utilisée, les cellules ne meurent pas. Cette propriété est évidemment très importante pour les applications qui vont être maintenant indiquées.

Sous un autre aspect, le procédé de l'invention est applicable à la fabrication de greffes. On sait en effet que, pour le traitement des brûlés, il est important de pouvoir utiliser la peau intact qui reste, afin d'éviter les problèmes posés par les défenses immunologiques du receveur en cas de greffe hétérologue. On préfère donc prélever sur les brûlés des parties de peau, afin de réaliser une autogreffe. Le tissu prélevé fait ensuite l'objet d'une séparation mécanique qui permet de multiplier deux à trois fois la surface couverte grâce au développement des cellules de la peau prélevée. Un perfectionnement important a été apporté à cette technique (voir IGEL H.J. FREEMAN A.E., BOECKMANN C.R. et KLEIFELD K.L. Arch. Surgery 1974, *108*, 724-729).

Selon la technique proposée par ces auteurs, des morceaux de peau sont prélevés sous forme de petits fragments en vue d'une autogreffe ultérieure. Le fractionnement des morceaux prélevés est effectué 3 à 5 semaines avant la greffe, et il convient donc de cultiver la peau prélevée. Le problème majeur est celui du transport des cellules épidermiques. A cet effet, on fait appel à des lambeaux de peau de porcs tués sur lesquels on met en place les fragments prélevés. Au bout d'une période d'environ 3 semaines, la peau de porc est couverte d'une culture d'épiderme. Cette technique est intéressante, car elle permet de prélever les morceaux de peau sur le patient lui-même. Par ailleurs, il n'y a aucune réaction indésirable vis-à-vis de la peau de porc. On obtient alors une surface couverte qui est de 20 à 40 fois la surface des fragments prélevés au départ.

Cette technique présente cependant des inconvénients. En effet, la partie du greffon qui est placée sur le tissu du patient est en contact avec celui-ci par l'intermédiaire de l'épiderme mort de la peau de porc portant l'épiderme cultivé. La présence de la peau de porc constitue un corps étranger indésirable. Un autre inconvénient est celui du fractionnement mécanique de la peau prélevée, car les fragments restent nécessairement assez gros. Il est souhaitable d'aboutir au fractionnement ultime, lequel, dans le cas idéal, est constitué par la cellule. On a donc récemment proposé une technique encore plus intéressante, dérivée de la technique de IGEL et FREEMAN précitée, selon laquelle on fractionne le greffon par un procédé enzymatique, 1 à 2 semaines avant la greffe. Ce temps est utilisé pour faire proliférer in vitro les cellules épidermiques. Pour assurer à ces cellules épidermiques cultivées un support qui permette leur prolifération in vitro, et ensuite leur transplantation, on a enlevé l'épiderme de lambeaux minces de peau humaine ou de peau de porc. Les lambeaux de derme obtenus ont été tués et conservés par congélation. L'avantage des lambeaux de derme désépidermisés est qu'ils portent leur « lamina densa », c'est-à-dire une couche de collagène sur laquelle les cellules épidermiques s'attachent et prolifèrent. Des greffons recombinés sont ainsi obtenus, formés de derme allo- ou xénogénique recouvert d'épiderme autologue. De tels greffons permettent de multiplier la surface épidermisée d'environ 80 fois en deux semaines. Une telle technique a été par exemple décrite dans un exposé présenté à l'occasion des 4èmes journées nationales des soins aux brûlés 21-22 Mai 1979 — à la Baule, France.

Quelle que soit la technique de greffe mise en œuvre, il importe d'augmenter la vitesse de prolifération des cellules épidermiques. On conçoit en effet l'intérêt d'effectuer des cultures importantes, mais surtout de pouvoir réaliser la culture dans un temps relativement court, par exemple inférieur ou égal à une semaine, pour permettre un traitement efficace du malade.

L'invention peut être appliquée avec avantage à une telle culture de cellules, puisque la surface couverte par la croissance des cellules épidermiques sera proportionnelle à l'activité du facteur de croissance RE. L'utilisation de l'extrait RE procure des avantages puisque, ainsi qu'il a été mentionné précédemment, il ne provoque pas d'effet mutagène. On constate également que les cellules épidermiques, après leur croissance en présence de RE, ne sont pas modifiées. Ainsi, le procédé de l'invention trouve une application intéressante dans la fabrication de greffons utilisables pour le traitement des brûlés.

En prélevant une petite surface de peau, par exemple de l'ordre de 2 à 3 cm$^2$, on peut obtenir une suspension de cellules épidermiques. Ainsi qu'on l'a mentionné précédemment, la culture peut être initiée et, après 8 jours environ, les colonies cellulaires sont suffisamment poussées. L'addition d'extrait rétinien RE pendant 2 jours consécutifs permet d'obtenir la synchronisation des mitoses.

Sous un autre aspect encore, l'invention peut recevoir des applications cosmétiques. Les agents cosmétiques conventionnels exercent un effet sur l'apparence de la partie du corps à traiter, par exemple la peau ou les cheveux. On constate cependant que les formulations cosmétiques ont de plus en plus tendance à contenir des agents actifs sur le métabolisme. En ce qui concerne l'application sur la peau, par exemple, on recherche des produits qui augmentent la croissance des cellules de l'épiderme, car on a constaté que ladite croissance conférait à la peau une apparence satinée améliorée. De même, on se préoccupe de mettre au point des produits favorisant la croissance des cheveux, lorsqu'ils sont appliqués sur la chevelure ou sur le cuir chevelu.

Selon l'invention, on peut donc introduire l'extrait RE directement dans une composition convenant à des applications cosmétiques. Bien entendu, cette composition devra être adaptée à l'application cosmétique recherchée, qu'il s'agisse par exemple de la régénération de la peau, de la lutte contre le vieillissement ou d'une application sur les cheveux. En outre, on peut utiliser l'extrait RE dans des applications plus spécifiques, en particulier pour le traitement de cellules épidermiques déficientes.

La plupart des agents actifs connus dans ce domaine sont inutilisables pour le traitement direct de la peau. Beaucoup d'entre eux sont tirés des euphorbes, et sont considérés comme des co-carcinogènes. Leur application généralisée est donc à écarter. Quant au facteur EGF, il apparaît comme inactif in vivo, ou, à tout le moins, son activité in vivo n'a pas encore été démontrée. Par ailleurs, son extraction est coûteuse. Les applications cosmétiques d'un tel produit à une large échelle ne sont donc pas prévisibles. On connaît également la propriété de la toxine du choléra d'activer fortement l'épiderme, mais un tel produit reste d'un emploi difficile.

Au contraire, selon l'invention, on peut utiliser l'extrait RE pour son activité sur les cellules de l'épiderme, en tirant profit de son absence de toxicité.

5

# 0 032 145

Ainsi, l'invention concerne-t-elle une composition cosmétique pharmaceutique, ou de diagnostic, caractérisée en ce qu'elle comprend, à titre d'agent actif, l'extrait RE. Une telle composition peut être appliquée par voie locale sur la partie du corps à traiter.

Pour les besoins de l'invention, on peut utiliser n'importe quel véhicule déjà proposé pour l'application sur la peau ou les cheveux. On peut notamment utiliser des compositions sous forme de lotions, gels, crèmes, pommades, etc... Des exemples de compositions convenant à l'application locale sont décrits par B. KAMMERAU et al. dans Arch. Derm. Res. 255 31-42 (1976).

La quantité de RE à mettre en œuvre dans les compositions de l'invention dépend de l'application envisagée. Bien entendu, l'application locale peut être renouvelée pour apporter la quantité d'agent actif nécessaire. En règle générale, des doses de 50 à 400 μg en protéines totales d'extrait RE par ml de culture de cellules épidermiques se sont avérées convenables. Des doses de 50 à 100 μg sont préférées.

L'invention est également applicable à un procédé de culture de longue durée de cellules épidermiques in vitro.

Les études concernant les cellules de la peau humaine adulte se heurtent à de nombreuses difficultés, surtout lorsqu'il s'agit de cellules d'épiderme humain adulte. Pour cette raison, la plupart des expériences sont réalisées sur des cultures de cellules non épidermiques spécialement fibroblastiques. Les fibroblastes sont connus pour posséder des propriétés de croissance en culture qui permettent des études pratiques. Cependant, la croissance des fibroblastes en culture est limitée, spécialement chez l'homme. Il existe, en effet, comme une programmation des fibroblastes humains qui les empêche de se dédoubler indéfiniment. D'autre part, on sait que le nombre de dédoublements des fibroblastes est plus important pour les cellules embryonnaires que pour les cellules adultes. Chez l'adulte, la culture meurt après une trentaine de dédoublements. Quelques cellules animales semblent échapper à cette loi, par exemple les cellules de souris, mais, chez l'homme, le seul moyen de modifier cette limitation naturelle de croissance est d'introduire dans la culture cellulaire des virus ou autres substances exogènes.

La limitation de croissance déjà observée chez les fibroblastes est encore plus nette pour les cellules épidermiques. La culture des cellules de l'épiderme de nouveau-né peut être dédoublée 4 à 5 fois, alors que les cellules issues de la peau adulte ne sont pratiquement pas « dédoublables ».

Le meilleur système actuel pour prolonger les cultures de longue durée de l'épiderme humain a été proposé dans l'article de RHEINWALD et GREEN cité supra. Il consiste à cocultiver des cellules épidermiques humaines avec des fibroblastes de souris 3T3 irradiées, servant de couche nourricière. Ces cellules irradiées ont la propriété de favoriser l'attachement des kératinocytes humains au cours des dédoublements de la culture, ainsi que de leur fournir des substances nourricières d'origine conjonctive augmentant leur croissance. L'irradiation subie bloque leur multiplication propre. De plus, les cellules 3T3 de souris ont un effet plutôt inhibiteur sur la croissance des fibroblastes humains, ce qui permet aux auteurs de contrôler la pureté de leurs cultures tout en partant de suspensions parfois fortement hétérogènes. Au cours des différents dédoublements de culture, les éventuels fibroblastes humains sont retirés par l'action de l'EDTA en même temps que les cellules de souris 3T3, les kératinocytes humains restant attachés au substrat pour être ensuite détachés enzymatiquement et mécaniquement. Le système de culture de longue durée peut recevoir l'adjonction de différents facteurs de croissance, c'est-à-dire des produits capables de stimuler la croissance des cellules, et en particulier celles des cellules extraites de tissus adultes. Ainsi, COHEN S., et ELLIOTT, G.A. dans la revue J. Invest. Dermatol. *401*-5 (1963) ont décrit un produit susceptible de favoriser la croissance des cellules de l'épiderme de souris. Des essais ont été réalisés en culture et les auteurs (RHEINWALD, J.G. and GREEN H. Nature 1977, *265,* 421-424) constatent que ce facteur de croissance est efficace sur les cultures cellulaires épidermiques de nouveau-né.

La question de l'effet activateur de l'EGF sur la croissance des cellules épidermiques adultes reste posée. En culture, et en présence de cellules de souris 3T3, une certaine activité a été rapportée sur les cellules humaines. Employé seul sur les cellules de cobaye, il s'est révélé inefficace. Enfin, chez le sujet entier, les preuves d'activation manquent.

D'autres activateurs ont été décrits et on peut, à titre de référence bibliographique, citer l'article de D. GOSPODAROWICZ dans Nature *249* (1974) 123. L'auteur décrit un facteur de croissance désigné par l'abréviation FGF, mais on ne trouve aucune indication sur l'activité de ce produit dans la croissance des cellules épidermiques humaines.

La toxine cholérique a aussi été étudiée dans les cultures de kératinocytes humains (GREEN H. Cell 1978, *15*801-811). Contrairement à son action sur beaucoup d'autres cellules étudiées, ce produit active fortement la croissance des kératinocytes humains, surtout des nouveau-nés. Cependant, il se lie fortement à la membrane des cellules et cette liaison est irréversible.

Selon l'invention, les cellules peuvent être directement cultivées in vitro sur un substrat, par exemple plastique, sans adjonction d'autres cellules. Le RE joue le rôle de facteur nourricier, son action plutôt négative sur les fibroblastes permettant à une culture, déjà purifiée au départ, de stabiliser à un nombre très faible la quantité de fibroblastes éventuellement contaminants. La longévité de la culture est ainsi accrue de manière très simple. De plus, l'activité certaine du RE sur les cellules adultes en fait un agent mitogène de choix pour le matériel qui ne réagit que faiblement au système de culture décrit précédemment avec des cellules de souris 3T3. Les études cytogénétiques décrites ci-dessus montrent de plus que ce produit n'a pas d'effet ni sur les chromosomes, ni sur les échanges de chromatides des

6

kératinocytes. Il n'est donc pas a priori suspect d'avoir des effets toxiques ni cancérigènes sur ces cellules.

Grâce à l'utilisation de RE, selon l'invention, les cultures de kératinocytes humains adultes peuvent ainsi être prolongées. Un tel procédé de culture est très simple à mettre en œuvre. Il respecte en outre la nature normale des kératinocytes humains adultes, leur fournissant uniquement un apport nutritif mitogène d'origine animale physiologique.

L'invention sera maintenant davantage illustrée sans être aucunement limitée par des expériences sur la culture in vitro de cellules épidermiques humaines adultes et par des exemples précis concernant les caryotypes et les échanges de chromatides sœurs sur les kératinocytes humains adultes en culture.

### Origine et préparation de la peau humaine

La peau humaine adulte est prélevée au cours d'interventions chirurgicales.

La peau mince, prélevée au dermatome électrique est placée directement dans un flacon contenant une solution d'antibiotiques (solution II, voir ci-après) et gardée à + 4 °C jusqu'à 5 jours.

La peau totale, non dégraissée, est placée dans un bocal contenant la solution II. Elle est conservée ainsi 24 heures. La peau est ensuite dégraissée au bistouri, et fixée solidement, derme en bas, sur une plaque de liège passée à l'alcool à 70°. La peau est ensuite coupée finement à l'aide d'un électrokératome réglé sur 0,4 mm d'épaisseur. Compte-tenu des variations d'épaisseur de l'épiderme suivant l'âge du donneur et la localisation du prélèvement, cette épaisseur de 0,4 mm correspond à l'épiderme total plus une partie du derme papillaire. La peau ainsi amincie est replacée dans la solution II et peut être conservée à + 4 °C jusqu'à 3 jours.

La peau mince et la peau totale ainsi préparées sont traitées de façon identique.

Les solutions de rinçage antibiotiques sont préparées dans 500 ml de milieu de base Eagle, comme suit :

Solution I : produit antibiotique antimycotique disponible sous la dénomination ABAM (GIBCO) : 20 ml Gentalline : 160 mg

Solution II : ABAM : 10 ml Gentalline : 80 mg

Solution III : ABAM : 5 ml.

Toutes les cultures ont été réalisées dans le milieu suivant : Dulbecco's Modification of Minimum Eagle Medium (Flow) en tampon Hépès, supplémenté à 20 % de sérum de veau fœtal. On a ajouté 1 % d'ABAM (Gibco) au milieu complet.

### Préparation des suspensions de kératinocytes à partir de la peau totale émincée.

La peau est passée successivement dans les trois solutions d'antibiotiques :

Solution I : 30 minutes
Solution II : 30 minutes
Solution III : 3 fois 10 minutes.

Entre chaque bain, elle est essorée sur un tampon de gaze stérile. La peau est ensuite stockée dans la solution III.

En vue du découpage, la peau est étalée, derme en bas, sur un couvercle de boîte de Pétri en verre stérile. A l'aide d'un bistouri stérile, elle est découpée en fragments d'environ 0,25 cm de côté.

Les fragments sont ensuite placés dans une solution de trypsine (Flow) à 0,25 % dans du tampon phosphate de Dulbecco sans calcium ni magnésium (PBS-Flow), à raison d'une vingtaine de fragments pour 5 ml de solution de trypsine, dans des tubes coniques de 50 ml (Falcon 2070). Les tubes sont alors placés au réfrigérateur à + 4 °C pendant une nuit. Le lendemain matin, les fragments sont récupérés à l'aide d'une grande pince stérile et placés sur un couvercle de boîte de Pétri en verre stérile. Ils sont étalés, épiderme contre le verre. Le derme et l'épiderme se séparent aisément avec deux pinces fines. Tous les fragments sont ainsi séparés, et placés dans un tube contenant 5 ml de PBS. Le couvercle est rincé avec 2 ml de PBS qui est ajouté dans le tube. Les tubes, une fois prêts, sont agités fortement à 80 tours/minute (Vortex), ce qui permet de libérer les cellules attachées à la surface du derme et de dissocier les couches inférieures de l'épiderme. Le PBS contenant derme + épiderme + cellules dissociées est versé dans un bécher à travers deux épaisseurs de gaze stérile, permettant de retenir le derme, l'épiderme et les gros aggrégats cellulaires.

Les suspensions récupérées ainsi sont replacées dans les tubes et centrifugées à 800 tours/minute pendant 10 minutes. Le PBS est rejeté et les culots cellulaires sont remis en suspension dans 4 ml de milieu complet par tube.

Le comptage des cellules est effectué après dilution au 1/2 d'un aliquote dans du Bleu Trypan, à l'aide d'un hématimètre de Burker.

### Etude de la stimulation des kératinocytes humains adultes par le RE

a) Essais à partir de 100 000 cellules par cm²

Les suspensions de cellules sont ajustées dans du milieu complet à 400 000 cellules par ml et réparties dans deux plaques à 24 puits de type Costar à raison de 0,5 ml de suspension par puits.

Les plaques sont mises à l'étude à 37 °C pendant 24 heures (jour 1) ; le milieu est renouvelé tous les deux jours et, dans une des deux plaques, 10 µl de RE, correspondant à 100 µg de protéines, sont ajoutés dans tous les puits, tous les jours. L'autre plaque sert de témoin. Le 8ème jour, le milieu de culture des deux plaques est rejeté, les cellules rincées au PBS et fixées par le fixateur de Carnoy (alcool méthylique : 3 volumes, acide acétique : 1 volume) pendant 20 minutes à la température ambiante, puis séchées à l'étuve à 37 °C pendant une heure. Les cultures sont alors colorées (Giemsa RAL : 4 volumes ; tampon phosphate Sorensen, pH 6,7 : 4 volumes ; eau déminéralisée 92 volumes) pendant 10 minutes, rincées à l'eau déminéralisée et laissées sur un papier filtre jusqu'à séchage complet.

b) Essais à partir d'un nombre de cellules variable

Les suspensions de cellules sont ajustées, dans du milieu complet à $10^5$, $2.10^5$, $4.10^5$, $8.10^5$, $12.10^5$, $16.10^5$ cellules par ml. Chaque dilution est répartie dans quatre puits de deux plaques dans un ordre croissant, à raison de 0,5 ml par puits.

Deux plaques sont ainsi remplies et mises à l'étuve à 37 °C. Les plaques sont alors traitées exactement comme les essais (a) précédents, l'une d'elles étant soumise à l'action du RE, l'autre servant de témoin. La fixation et la coloration, le 8ème jour, sont réalisées de manière identique.

La surface colorée visible dans les puits et l'intensité de la coloration indiquent l'intensité de la croissance des cellules. Pour quantifier cette croissance et pour vérifier qu'il ne s'agissait pas d'un meilleur étalement des cellules, on a effectué un comptage de mitoses.

Dans chaque puits de chaque plaque, on a compté le nombre de mitoses présentes dans cinq champs microscopiques à un grossissement × 250. On a ainsi déterminé le nombre de mitoses par champ pour chaque dilution de cellules au départ, et on a comparé les résultats entre les cultures traitées par le RE et les cultures témoins.

La pureté des cultures a été vérifiée en les soumettant à la réaction à la leucine aminopeptidase (LAP) (voir article de NACHLAS et coll. cité supra). On a trouvé que cette réaction était négative, alors qu'elle est positive dans les cellules non épidermiques.

### Préparation des étalements chromosomiques

Les cultures pour études cytogénétiques ont été faites sur lamelles de verre dans des tubes de Leighton.

Les suspensions de kératinocytes ont été ajustées à une concentration de 400 000 cellules par ml dans du milieu complet et réparties à raison de 2 ml par tube dans des tubes de Leighton dont le méplat contenait une lamelle de verre. Les tubes ont été ensuite bouchés et placés à l'étuve à 37 °C horizontalement.

Le milieu est renouvelé après 48 heures et après 5 jours de culture.

Au 8ème jour d'incubation, 40 µl (400 µg de protéines) de RE sont ajoutés dans les tubes. Le 9ème jour, le milieu est rejeté et remplacé par du milieu frais ; 40 µl de RE sont encore ajoutés. Huit heures après la dernière addition de RE, les cultures sont traitées par un sel de dicolcine disponible sous la dénomination « Colcemid » (Gibco) à une concentration finale de $0,25.10^6$ M durant 15 heures.

Les mitoses stimulées par le RE et bloquées en métaphases par l'action de la « Colcemid » sont préparées sur lamelle.

Après 15 heures de contact avec la « Colcemid », les lamelles sont rincées au PBS, retirées des tubes et placées dans des boîtes de Pétri de 6 cm de diamètre (Falcon 1007), afin de suivre l'évolution des cellules au cours du traitement sous un microscope inversé à contraste de phase.

Les lamelles sont recouvertes par 2 ml de milieu hypotonique, ayant la composition ci-après :

KCl 0,075 M : 25 vol.
$H_2O$ distillée : 25 vol.
Hyaluronidase injectable (Choay) : 3 vol.

Cette solution est préchauffée 30 minutes à 37 °C. Les boîtes contenant les lamelles sont placées à l'étuve à 37 °C pendant 30 minutes. La hyaluronidase permet au milieu aqueux de pénétrer à l'intérieur des cellules en mitose et de les faire gonfler. Les boîtes sont alors retirées de l'étuve et le fixateur suivant est ajouté :

alcool méthylique absolu : 3 volumes
acide acétique glacial : 1 volume.

Cette solution est ajoutée goutte à goutte, très lentement pour atteindre dans les boîtes, en une heure environ, la dilution 1 : 1 dans le milieu hypotonique.

Les boîtes sont alors replacées à l'étuve à 37 °C pendant 10 minutes. Le mélange milieu hypotonique-fixateur est alors retiré des boîtes et remplacé par du fixateur pur pendant 20 minutes.

Cette fixation très lente permet à la solution alcoolique de remplacer peu à peu l'eau à l'intérieur des cellules gonflées.

Les lamelles sont sorties des boîtes, posées sur un portoir métallique, séchées à l'étuve à 37 °C pendant une heure et colorées au Giemsa pendant 10 minutes.

Giemsa (RAL) : 4 volumes
Tampon phosphate Sorensen, pH 6,7 : 4 volumes
$H_2O$ distillée : 92 volumes.

Pour obtenir des bandes R sur les préparations, on a utilisé la technique de dénaturation par la chaleur (DUTRILLAUX et coll. 1972, CHARPENTIER et coll. 1972). Pour cela, les lamelles non colorées sont mises dans un tube contenant une solution saline de Earle pH 6,5 à 87 °C. Les tubes sont maintenus au bain-marie à cette température pendant un temps variant de manière inversement proportionnelle à l'âge des préparations. Pour des préparations de 15 jours, le temps est d'une trentaine de minutes.

Les lamelles sont alors sorties des tubes, rincées à l'eau distillée et colorées avec le colorant de Giemsa utilisé précédemment.

Après 10 minutes de coloration, les lamelles sont rincées à l'eau déminéralisée, essorées entre deux papiers-filtres, et laissées à sécher à l'air pendant deux heures.

Le montage des lamelles est fait au DPX (GURR), sur des lames d'histologie.

Préparation des échanges de chromatides

La préparation des cultures et la mise en route sont identiques à celles décrites pour les préparations chromosomiques. Au 8ème jour, le milieu de culture est rejeté et remplacé par du milieu frais contenant de la bromodeoxyuridine (BUdR/Sigma), à une concentration finale de 0,5 µg par ml. Quarante µl (400 µg de protéines) de RE sont ajoutés dans chaque tube et ceux-ci sont remis à l'étuve à 37 °C. Le 9ème jour, le milieu est rejeté et remplacé par du milieu contenant aussi du BUdR à la même concentration. La même quantité de RE est ajoutée. Dix huit heures après, les cultures sont traitées par la Colcemid (Gibco) : $0,25 \cdot 10^6$ M comme précédemment, et ceci pendant 15 heures.

La préparation des métaphases est identique à celle décrite précédemment.

Le BUdR est incorporé pendant deux cycles consécutifs à la place de la thymidine dans l'ADN d'une majorité de cellules, stimulées par le RE. Les chromosomes métaphasiques, après le deuxième cycle de replication, présentent une chromatide bisubstituée par le BUdR et une chromatide mono-substituée. Cette dernière fixe le fluorochrome Hoechts 33258 que l'on remplace ensuite par du Giemsa. La technique utilisée est dérivée de celle de DE WEERDKASTELEIN et coll. (1977), elle-même adaptée de la méthode décrite par PERRY et col. (1974). Les lamelles sont plongées dans une solution de fluorochrome Hoechst 33258 (Hoechst) à 0,5 µg par ml de PBS pendant 15 minutes. Elles sont ensuite placées, cellules au-dessus, dans des boîtes de Pétri de 6 cm de diamètre contenant un papier-filtre humecté de PBS, sous une lampe à U. V. (254 nm) pendant une nuit.

Les cellules sont alors placées au bain-marie à 61 °C pendant une heure, dans des tubes contenant du tampon :

KCl : 0,3 M
Citrate de sodium : 0,03 M.

Les lamelles sont alors rincées à l'eau distillée, colorées au Giemsa, séchées et montées comme précédemment.

Les observations et comptages de chromosomes et d'échanges de chromatides ont été faits avec un microscope orthoplan à objectif à immersion.

Exemple 1

Augmentation de la densité cellulaire et de la surface couverte par des kératinocytes épidermiques humains adultes après traitement par l'extrait rétinien (RE)

Les résultats de cet exemple sont illustrés à la figure 1.

Dans cette expérience représentative, un spécimen de 2 à 3 cm² de peau a été prélevé dans la région thoracique d'une femme de 30 ans. Ce spécimen a été recoupé en morceaux amincis de 2 à 4 mm². Ces fragments ont été incubés une nuit dans de la trypsine à 0,25 % dans du tampon salin au phosphate (PBS), à + 4 °C. Une fois dissociés, l'épiderme et le derme ont été doucement agités dans du PBS pour récolter les cellules. Après filtration à travers deux épaisseurs de gaze, les cellules ont été sédimentées à 800 t.p.m. pendant 10 minutes. Le milieu de culture (T.C.M.) consistait en la modification de Dulbecco du MEM de Eagle, en tampon Hépès, supplémenté avec 20 % de sérum de veau fœtal. Les plaques de culture de tissu (COSTAR-3524) à 24 puits (2 cm² par puits) comme il est montré sur la figure 1 ont été ensemencées à des concentrations cellulaires variées.

Pour la préparation du RE on a utilisé des rétines de bovins et on a opéré comme suit :

Les yeux de bœufs sont prélevés à l'abattoir quelques instants après la mort de l'animal. Ils sont alors transportés sur la glace et la dissection a lieu dans les 3 heures qui suivent. Après nettoyage du globe par un jet d'alcool à 70 %, la cornée est découpée et le cristallin, l'iris, et l'humeur vitrée sont recueillis dans des récipients séparés, sur glace. La rétine est alors retirée à l'aide d'une pince et lavée dans une solution isotonique tampon neutre. Puis les rétines sont rassemblées.

Toutes les opérations consécutives d'extraction du facteur RE sont réalisées à froid.

50 rétines sont rassemblées dans 50 ml de tampon PBS, puis elles sont broyées dans un Potter. Une première centrifugation a lieu à 11 000 tours/minute pendant 30 minutes. Le surnageant est alors directement filtré sur une succession de filtres « millipore » avec des diamètres de pores décroissants : 3 μ, 1,2 μ, 0,45 μ, 0,22 μ, le dernier amenant la stérilisation. Le facteur RE est conservé congelé.

On peut obtenir également l'extrait RE avec des rendements analogues en opérant :

1) une centrifugation supplémentaire à 100 000 g avant les filtrations.

2) une dialyse contre le tampon PBS pendant une nuit suivie de la filtration pour stérilisation.

La solution protéique a été ajustée à 5 mg par ml en PBS. Le contenu protéique a été mesuré selon la technique décrite par BRADFORD M.M. Analytical Biochem. *72*, 248-254 (1976) en utilisant le sérum albumine bovine (fraction V) comme standard. 20 μl par puits (0,5 ml de TCM) ont été ajoutés chaque jour, de la manière suivante : A 24 heures, le RE a été ajouté, le TCM a été enlevé le 2ème jour et remplacé par du TCM + RE. Au jour 3, le RE a été ajouté sans changement du TCM. Au jour 4, le TCM a été enlevé et remplacé par du TCM frais + RE. Aux jours 5 et 7, les cultures ont été traitées comme au jour 3, aux jours 6 et 8, comme au jour 4. Les témoins ont été traités de façon similaire mais le RE a été omis.

La figure 1 montre une coloration au Giemsa de culture 8 jours après l'ensemencement.

De gauche à droite, les cellules ont été ensemencées à

a) $5 \times 10^4$
b) $10^5$
c) $2 \times 10^5$
d) $4 \times 10^5$
e) $6 \times 10^5$
f) $8 \times 10^5$

cellules par puits. L'effet du RE sur la surface couverte par les cellules épidermiques est clairement visible dans les rangées a), b) et c). L'augmentation de la densité cellulaire peut se voir aussi dans les rangées c), d), e) et f).

## Exemple 2

Augmentation de l'activité mitotique résultant du traitement par le RE

Les résultats de cet exemple sont illustrés à la figure 2.

Les figures mitotiques ont été comptées dans des cultures montrées sur la figure 1. Pour ce faire, 5 champs microscopiques ont été examinés au grossissement 250, dans chaque puits. Sur la figure 2, le nombre de figures mitotiques par champ microscopique (ordonnées) est porté en fonction de la concentration de cellules ensemencées par puits (abscisses). Chaque point de la courbe représente la valeur moyenne de 12 comptes (3 champs microscopiques × 4 puits). On peut voir que, dans les cultures témoins, environ 3 figures mitotiques peuvent être détectées dans un champ microscopique pour une concentration d'ensemencement de 4, 6, 4 et $8 \times 10^5$.

Dans les cultures traitées par le RE, le nombre de figures mitotiques est au moins doublé.

## Exemple 3

Accumulation de mitoses pour caryotypes

Les résultats de cet exemple sont illustrés à la figure 3.

Pour les caryotypes, des spécimens de peau de 6 sujets adultes ont été prélevés. Les suspensions cellulaires ont été préparées comme il est indiqué à l'exemple 1, ajustées à $2 \times 10^5$ cellules par ml dans du TCM. On a ensemencé 2 ml de ces suspensions sur des lamelles de 11 × 22 mm (environ 2 cm²) en verre dans des tubes de Leighton. Les 8ème et 9ème jours, 100 μg (équivalent protéine) par ml de RE ont été ajoutés dans chaque tube. 12 heures après la 2ème addition de RE, les cellules ont été traitées à la Colcemid ($0,25 \times 10^5$ M) pendant 15 heures pour accumuler les métaphases. Les lamelles ont été rincées au PBS et transférées dans des boîtes à moitié remplies de solution hypotonique (KCl 0,075 M : $H_2O$ : 1 :

1) à laquelle était ajouté 6 % de hyaluronidase (Choay). Après 30 minutes à + 37 °C, le fixateur (méthanol, acide acétique, 3 : 1) a été ajouté à la solution hypotonique, très lentement, goutte à goutte, pour atteindre une dilution 1 : 1 en environ 1 heure. Ce mélange a été remplacé après 10 minutes à + 37 °C par du fixateur pur pendant 30 minutes à la température ambiante.

La figure 3 est une vue en contraste de phase de 6 mitoses gonflées dans un seul champ, après addition des premières gouttes de fixateur. Après la fixation, les lamelles ont été séchées à + 37 °C pendant une heure, colorées au Giemsa, et montées au DPX (GURR).

Exemple 4 ·

Echanges de chromatides sœurs sur kératinocytes humains adultes.

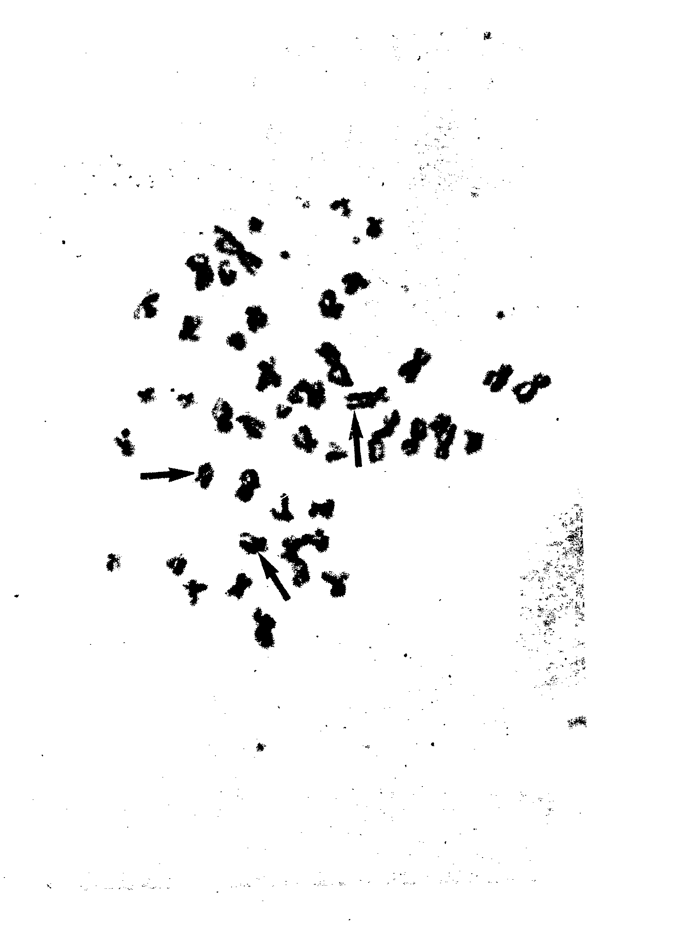

Les résultats de cet exemple sont illustrés à la figure 4.

Des cultures de kératinocytes humains adultes (4 expériences) ont été débutées à la concentration de $4 \times 10^5$ cellules par lamelle de verre en tube de Leighton, comme dans l'exemple 3. Les cellules ont été cultivées pendant 8 jours dans du TCM normal, comme dans l'exemple 1, sans RE. Le 8ème jour, le TCM a été rejeté et remplacé par du TCM contenant du BUdR (concentration finale 0,2 µg par ml). 100 µg (équivalent protéine) de RE ont été ajoutés. Après 24 heures à + 37 °C, le milieu a été rejeté et remplacé par du TCM frais contenant la même quantité de BUdR et de RE respectivement.

Les cellules ont été incubées ainsi pendant 36 heures. Après traitement par la Colcemid, la solution hypotonique et le fixateur, comme il est indiqué sur la figure 3, les cultures ont été colorées au Hoechst 33 358 (0,5 µg par ml en PBS) pendant 15 minutes. Puis, après une exposition d'une nuit à la lumière U. V. à 254 nm, les lamelles ont été traitées au SSC × 2 (0,3 M KCl 0,03 M citrate de sodium) à + 61 °C pendant une heure (DE WEERD-KASTELEIN E.A., KEIZER W., RAINALDI G. et BOOTSMA D. Mutat. Res. *45* 253-261 (1977)).

Les cellules ont été rincées à l'eau et colorées au Giemsa à 4 % en tampon phosphate, pH 6,7 pendant 10 minutes.

La figure 4 a été prise à l'objectif × 100 à immersion, sur une pellicule Kodak Microfile. Les flèches indiquent les échanges de chromatides sœurs spontanés.

Exemple 5

Comparaison de l'activité du RE et d'un autre facteur de croissance (FGF)

L'exemple ci-après a pour but de montrer la différence d'activité sur la croissance des myoblastes du RE et du facteur FGF (voir article de D. Gospodarowicz cité supra).

Des myoblastes préparés par dissociation de muscles de membres de veau sont ensemencés dans des boîtes de Pétri. Le milieu de culture ne contient que 0,5 % de sérum. Pendant 2 jours, les cellules reçoivent 50 µl de RE ou 10 ng de FGF. Le nombre de cellules est déterminé directement sur la moitié des boîtes ; sur l'autre moitié, l'incorporation de thymidine radioactive est mesurée après 4 heures d'incubation. Les résultats montrent que le témoin contient 40 000 cellules et 8 100 Cpm, alors que l'échantillon traité au RE contient 120 000 cellules et 41 000 Cpm et l'échantillon traité au FGF 40 000 cellules et 25 000 Cpm.

Il y a donc une forte stimulation due au RE. Le FGF, dans ce cas, ne modifie pas le nombre de cellules par rapport au témoin.

Exemple 6

Cet exemple concerne l'action du facteur RE sur la croissance de cellules épidermiques de cobaye en culture.

Les cellules étaient issues de l'oreille de cobaye Hartley adulte (classe IV). Trois types de cellules ont été isolées :
a) cellules épidermiques basales,
b) cellules épidermiques suprabasales,
c) cellules du tissu conjonctif dermique.

Les cellules épidermiques ont été ensemencées à trois concentrations différentes : 500 000/cm², 250 000/cm² et 100 000/cm².

Les cellules fibroblastiques étaient ensemencées à 50 000/cm² et à 400 000/cm².

Toutes les cultures ont été faites en milieu de Eagle BME + 10 % de sérum de veau fœtal. Les cellules étaient cultivées en triplicate dans des boîtes de Pétri en matière plastique.

L'extrait RE a été ajouté au milieu de culture à la dose de 100 µg d'équivalent protéine par ml. Les cellules ont été ensemencées dans un milieu de culture contenant le RE. Les milieux ont été changés aux 1er, 3ème, 5ème et 8ème jours et remplacés par des milieux frais contenant également la même dose de RE. Les cellules ont donc été maintenues en présence de RE pendant toute la durée de l'expérience.

De la thymidine tritiée ($^3$H-méthyl-thymidine Amersham a.s. 40 Ci/mM) était ajoutée à la concentration finale de 5 $\mu$Ci/ml.

Après une heure d'incubation, le milieu était rejeté, les cellules étaient lavées une fois au PBS puis dans la potasse 0,3 M directement dans la boîte de Pétri pendant une heure à 37 °C. La solution visqueuse obtenue était précipitée avec du PCA à 70 %. Le précipité était lavé trois fois avec du PCA 0,4 M et hydrolysé dans du PCA 0,5 M 15 minutes à 95 °C. Un aliquote de surnageant était compté pour sa radioactivité et un autre aliquote était utilisé pour mesurer sa teneur en DNA selon la technique de Burton.

En vue de l'estimation de la désquamation, aux jours 1, 3, 5 et 8, les milieux de culture des cellules étaient récupérés et filtrés sur filtre Millipore pesé de 0,45 $\mu$. Après 48 heures, les filtres séchés à l'air étaient repesés. Le poids sec obtenu correspondait aux taux de protéines en mg.

## Résultats

Les essais rapportés ci-dessus montrent que l'extrait RE a un effet significatif sur la synthèse de DNA par les cellules basales au 8ème jour à $2 \times 10^6$ cellules, soit à la dose de 250 000 cellules par cm$^2$. L'effet est moins constant sur les mêmes cellules à 500 000 par cm$^2$.

L'effet d'induction est surtout net au 8ème jour sur les cellules matures (suprabasales) ensemencées au taux de 250 000/cm$^2$.

Enfin, on constate un effet nul à 8 jours sur les cellules du derme (essentiellement fibroblastes).

En outre, la désquamation n'est pas modifiée.

Au total, l'extrait RE s'est révélé activateur dans les cellules épidermiques (basales + suprabasales) ensemencées à 250 000 par cm$^2$ après 8 jours d'exposition des cellules au produit. Dans les mêmes conditions, l'action était nulle pour les cellules dermiques.

On voit donc que l'extrait RE exerce une action stimulante de la croissance in vitro des cellules épidermiques adultes aussi bien chez l'homme que chez le cobaye.

## Exemple 7

Des expériences complémentaires ont été réalisées pour montrer l'absence d'effets secondaires de l'extrait RE.

L'étude d'activité mutagène du RE sur différentes souches de *Salmonella typhimurium* et aux concentrations de 22,5 à 720 $\mu$g/culture, n'a montré aucun effet mutagène selon la méthode d'Ames.

A la concentration de 100 $\mu$g/ml, l'extrait RE n'a manifesté aucune activité d'irritation locale sur la peau.

L'indice d'irritation sur la conjonctive oculaire, à la concentration de 5 mg/ml, n'a montré également aucune activité irritante pour l'œil du lapin.

**Revendications** (pour les Etats contractants : CH, DE, FR, GB, LI, LU, NL, SE)

1. Procédé pour stimuler la croissance in vitro des cellules d'épiderme, en particulier adulte, caractérisé en ce qu'on met en contact les cellules de l'épiderme avec un extrait (RE) salin aqueux de tissu oculaire.

2. Procédé selon la revendication 1, caractérisé en ce que l'agent actif est extrait de tissus de l'œil tels que la choroïde, l'iris et l'humeur vitrée.

3. Procédé selon l'une des revendications 1 ou 2, caractérisé en ce que l'extrait RE est obtenu par traitement de tissus oculaires de bovins par une solution saline aqueuse tamponnée à un pH voisin de 7,2.

4. Procédé selon l'une quelconque des revendications 1 à 3, caractérisé en ce qu'on utilise 50 à 400 $\mu$g, en protéine totale, d'extrait par ml de culture de cellules épidermiques, des doses de 50 à 100 $\mu$g étant préférées.

5. Procédé selon l'une quelconque des revendications 1 à 4, caractérisé en ce qu'on cultive des cellules d'épiderme humain.

6. Application du procédé selon l'une quelconque des revendications 1 à 5 à l'étude des chromosomes de la cellule de l'épiderme humain, en particulier pour la détection des anomalies chromosomiques naturelles ou induites.

7. Application selon la revendication 6 pour l'évaluation des échanges de chromatides sœurs (SCE) dans des cultures in vitro de kératinocytes humains adultes.

8. Application selon l'une des revendications 6 ou 7, caractérisée en ce qu'on effectue une mise en culture préalable normale des cellules épidermiques en l'absence de facteur de croissance RE, en ce que, après une période comprise entre 5 et 10 jours, par exemple au bout de 8 jours, on introduit dans la culture une quantité efficace de RE, et en ce que, un ou deux jours après, on introduit une autre quantité efficace de RE, en vue d'obtenir une seconde replication des cellules.

9. Application selon l'une des revendications 6 ou 7, caractérisée en ce qu'on effectue la mise en culture des cellules épidermiques en introduisant, à l'origine, une quantité efficace de RE, et en ce qu'on arrête ensuite la culture pour permettre la synchronisation des cellules.

10. Application du procédé selon l'une quelconque des revendications 1 à 5 à la fabrication de greffes, et spécialement d'auto-greffes, utilisables chez l'homme, par exemple en vue du traitement des brûlés.

11. Application selon la revendication 10 intégrée à une technique de greffage consistant à prélever de petits fragments de peau en vue de l'auto-greffe ultérieure, à fractionner le greffon par des moyens enzymatiques 1 à 2 semaines avant la culture, à faire proliférer les cellules épidermiques cultivées sur un support constitué de lambeaux minces de peau humaine ou de peau de porc, l'extrait RE étant ajouté en quantité efficace en vue d'une culture accélérée des cellules épidermiques, et à réaliser la greffe de manière classique.

12. Greffon mis en œuvre ou obtenu dans l'application selon l'une des revendications 10 ou 11.

13. Composition cosmétique, pharmaceutique ou de diagnostic comprenant, à titre d'agent actif l'extrait RE, et présentée en vue de l'application par voie locale sur la partie du corps à traiter.

14. Application du procédé selon l'une quelconque des revendications 1 à 5, à la culture de longue durée des cellules épidermiques.

15. Application selon la revendication 14 dans laquelle on cultive directement les cellules sur un substrat, par exemple plastique, en présence d'une quantité efficace de l'extrait RE, et sans adjonction d'autres cellules.


**Revendications** (pour l'Etat contractant AT)

1. Procédé pour stimuler la croissance in vitro des cellules d'épiderme, en particulier adulte, caractérisé en ce qu'on met en contact les cellules de l'épiderme avec un extrait (RE) salin aqueux de tissu oculaire.

2. Procédé selon la revendication 1, caractérisé en ce que l'agent actif est extrait de tissus de l'œil tels que la choroïde, l'iris et l'humeur vitrée.

3. Procédé selon l'une des revendications 1 ou 2, caractérisé en ce que l'extrait RE est obtenu par traitement de tissus oculaires de bovins par une solution saline aqueuse tamponnée à un pH voisin de 7,2.

4. Procédé selon l'une quelconque des revendications 1 à 3, caractérisé en ce qu'on utilise 50 à 400 μg, en protéine totale, d'extrait par ml de culture de cellules épidermiques, des doses de 50 à 100 μg étant préférées.

5. Procédé selon l'une quelconque des revendications 1 à 4, caractérisé en ce qu'on cultive des cellules d'épiderme humain.

6. Procédé selon l'une quelconque des revendications 1 à 5, caractérisé en ce qu'on étudie les chromosomes de la cellule de l'épiderme humain, en particulier pour la détection des anomalies chromosomiques naturelles ou induites.

7. Procédé selon la revendication 6, caractérisé en ce qu'on évalue des échanges de chromatides sœurs (SCE) dans des cultures in vitro de kératinocytes humains adultes.

8. Procédé selon l'une quelconque des revendications 1 à 7, caractérisé en ce qu'on effectue une mise en culture préalable normale des cellules épidermiques en l'absence de facteur de croissance RE, en ce que, après une période comprise entre 5 et 10 jours, par exemple au bout de 8 jours, on introduit dans la culture une quantité efficace de RE, et en ce que, un ou deux jours après, on introduit une autre quantité efficace de RE, en vue d'obtenir une seconde replication des cellules.

9. Procédé selon l'une quelconque des revendications 1 à 7, caractérisé en ce qu'on effectue la mise en culture des cellules épidermiques en introduisant, à l'origine, une quantité efficace de RE, et en ce qu'on arrête ensuite la culture pour permettre la synchronisation des cellules.

10. Procédé selon l'une quelconque des revendications 1 à 9, caractérisé en ce qu'on fabrique des greffes et spécialement des auto-greffes utilisables chez l'homme, par exemple en vue du traitement des brûlés.

11. Procédé selon la revendication 10, caractérisé en ce qu'on l'intègre à une technique de greffage consistant à prélever de petits fragments de peau en vue de l'auto-greffe ultérieure, à fractionner le greffon par des moyens enzymatiques 1 à 2 semaines avant la culture, à faire proliférer les cellules épidermiques cultivées sur un support constitué de lambeaux minces de peau humaine ou de peau de porc, l'extrait RE étant ajouté en quantité efficace en vue d'une culture accélérée des cellules épidermiques, et à réaliser la greffe de manière classique.

12. Procédé selon l'une quelconque des revendications 1 à 11, caractérisé en ce qu'on réalise une culture de longue durée de cellules épidermiques.

13. Procédé selon l'une quelconque des revendications 1 à 14, caractérisé en ce qu'on cultive directement les cellules sur un substrat, par exemple plastique, en présence d'une quantité efficace de l'extrait RE, et sans adjonction d'autres cellules.

14. Procédé pour la préparation d'une composition cosmétique, pharmaceutique ou de diagnostic, caractérisé en ce qu'on mélange l'extrait RE obtenu par le procédé selon l'une des revendications 1 à 13 à un véhicule adapté à l'application par voie locale sur la partie du corps à traiter.

**Claims** (for the Contracting States : CH, DE, FR, GB, LI, LU, NL, SE)

1. Process for stimulating in vitro growth of epidermis cells, in particular adult epidermis cells, characterised in that the epidermis cells are brought into contact with an aqueous saline extract (RE) of ocular tissue.

2. Process according to Claim 1, characterised in that the active agent is extracted from eye tissue such as the choroid, the iris and the vitreous humour.

3. Process according to either of Claims 1 and 2, characterised in that the RE extract is obtained by treatment of ocular tissue from cattle with an aqueous saline solution buffered at a pH of about 7.2.

4. Process according to any one of Claims 1 to 3, characterised in that 50 to 400 μg, in total protein, of extract are used per ml of epidermal cell culture, doses of 50 to 100 μg being preferred.

5. Process according to any one of Claims 1 to 4, characterised in that human epidermis cells are cultured.

6. Use of the process according to any one of Claims 1 to 5 in the study of chromosomes of the human epidermis cells, in particular for the detection of natural or induced chromosomal anomalies.

7. Use according to Claim 6 for evaluating sister chromatid changes (SCC) in cultures, in vitro, of adult human keratinocytes.

8. Use according to either of Claims 6 and 7, characterised in that epidermal cells are first subjected to normal culture in the absence of the growth factor RE, in that, after a period of between 5 and 10 days, for example after 8 days, an effective amount of RE is introduced into the culture, and in that, one or two days thereafter, another effective amount of RE is introduced in order to obtain a second replication of the cells.

9. Use according to either of Claims 6 and 7, characterised in that the epidermal cells are cultured by introducing, at the start, an effective amount of RE, and in that the culture is then stopped in order to permit synchronisation of the cells.

10. Use of the process according to any one of Claims 1 to 5 for the preparation of grafts, in particular autografts, which can be used on humans, for example for the treatment of burns.

11. Use according to Claim 10 incorporated into a grafting technique consisting of removing small fragments of skin for the subsequent autograft, fractionating the graft by enzymatic means 1 to 2 weeks before the culture, causing the cultured epidermal cells to proliferate on a support consisting of thin pieces of human skin or pig skin, the RE extract being added in an effective amount for accelerated culture of the epidermal cells, and grafting in the conventional manner.

12. Grafts used or obtained in the use according to either of Claims 10 and 11.

13. Cosmetic, pharmaceutical or diagnostic composition containing, as the active agent, RE extract and formulated for local application to the part of the body to be treated.

14. Use of the process according to any one of Claims 1 to 5 for prolonged culture of epidermal cells.

15. Use according to Claim 14, in which the cells are cultured directly on a substrate, for example a plastic, in the presence of an effective amount of RE extract and without the addition of other cells.

**Claims** (for the Contracting State AT)

1. Process for stimulating in vitro growth of epidermis cells, in particular adult epidermis cells, characterised in that the epidermis cells are brought into contact with an aqueous saline extract (RE) of ocular tissue.

2. Process according to Claim 1, characterised in that the active agent is extracted from eye tissue such as the choroid, the iris and the vitreous humour.

3. Process according to either of Claims 1 and 2, characterised in that the RE extract is obtained by treatment of ocular tissue from cattle with an aqueous saline solution buffered at a pH of about 7.2.

4. Process according to any one of Claims 1 to 3, characterised in that 50 to 400 μg, in total protein, of extract are used per ml of epidermal cell culture, doses of 50 to 100 μg being preferred.

5. Process according to any one of Claims 1 to 4, characterised in that human epidermis cells are cultured.

6. Process according to any one of Claims 1 to 5, characterised in that chromosomes of the human epidermis cells are studied, in particular for the detection of natural or induced chromosomal anomalies.

7. Process according to Claim 6, characterised in that sister chromatid changes (SCC) in cultures, in vitro, of adult human keratinocytes are evaluated.

8. Process according to any one of Claims 1 to 7, characterised in that epidermal cells are first subjected to normal culture in the absence of the growth factor RE, in that, after a period of between 5 and 10 days, for example after 8 days, an effective amount of RE is introduced into the culture, and in that, one or two days thereafter, another effective amount of RE is introduced in order to obtain a second replication of the cells.

9. Process according to any one of Claims 1 to 7, characterised in that the epidermal cells are cultured by introducing, at the start, an effective amount of RE, and in that the culture is then stopped in order to permit synchronisation of the cells.

**0 032 145**

10. Process according to any one of Claims 1 to 9, characterised in that grafts, in particular autografts, which can be used on humans, for example for the treatment of burns, are prepared.

11. Process according to Claim 10, characterised in that it is incorporated into a grafting technique consisting of removing small fragments of skin for the subsequent autograft, fractionating the graft by enzymatic means 1 to 2 weeks before the culture, causing the cultured epidermal cells to proliferate on a support consisting of thin pieces of human skin or pig skin, the RE extract being added in an effective amount for accellerated culture of the epidermal cells, and grafting in the conventional manner.

12. Process according to any one of Claims 1 to 11, characterised in that prolonged culture of epidermal cells is carried out.

13. Process according to any one of Claims 1 to 14, characterised in that the cells are cultured directly on a substrate, for example a plastic, in the presence of an effective amount of RE extract and without the addition of other cells.

14. Process for the preparation of a cosmetic, pharmaceutical or diagnostic composition, characterised in that RE extract obtained by the process according to any one of Claims 1 to 13 is mixed with a vehicle suitable for local application to the part of the body to be treated.


**Ansprüche** (für die Vertragsstaaten : CH, DE, FR, GB, LI, LU, NL, SE)


1. Verfahren zum Anregen des Wachstums in vitro von Epidermiszellen, insbesondere ausgewachsenen, dadurch gekennzeichnet, daß man die Zellen der Epidermis mit einem wäßrigen Salzextrakt (RE) von Augengewebe in Berührung bringt.

2. Verfahren nach Anspruch 1, dadurch gekennzeichnet, daß das aktive Mittel von Geweben des Auges wie beispielsweise der Gefäßhaut des Auges, der Regenbogenhaut und der Glaskörperflüssigkeit, extrahiert wird.

3. Verfahren nach einem der Ansprüche 1 oder 2, dadurch gekennzeichnet, daß der Extrakt RE durch Behandlung von Augengeweben von Rindern mit einer bei einem pH-Wert nahe 7,2 gepufferten wäßrigen Salzlösung erhalten wird.

4. Verfahren nach irgendeinem der Ansprüche 1 bis 3, dadurch gekennzeichnet, daß man 50 bis 400 µg, als Gesamtprotein, Extrakt pro ml Epidermiszellkultur verwendet, wobei Dosen von 50 bis 100 µg bevorzugt werden.

5. Verfahren nach irgendeinem der Ansprüche 1 bis 4, dadurch gekennzeichnet, daß man menschliche Epidermiszellen kultiviert.

6. Anwendung des Verfahrens nach irgendeinem der Ansprüche 1 bis 5 auf die Untersuchung der Chromosomen der menschlichen Epidermiszelle, insbesondere zur Entdeckung von natürlichen oder induzierten Chromosomenanomalien.

7. Anwendung nach Anspruch 6 auf die Bewertung der Austausche von Schwesterchromatiden (SCE) in in-vitro-Kulturen von ausgewachsenen menschlichen Keratinozyten.

8. Anwendung nach einem der Ansprüche 6 oder 7, dadurch gekennzeichnet, daß man Epidermiszellen in Abwesenheit des Wachstumsfaktors RE in eine vorherige normale Kultur bringt, daß man nach einer Zeit zwischen 5 und 10 Tagen, beispielsweise nach 8 Tagen, in die Kultur eine wirksame RE-Menge einführt und daß man einen oder zwei Tage danach eine weitere wirksame RE-Menge einführt, um eine zweite Replikation der Zellen zu erhalten.

9. Anwendung nach einem der Ansprüche 6 oder 7, dadurch gekennzeichnet, daß man Epidermiszellen in Kultur bringt, indem man anfangs eine wirksame RE-Menge einführt und anschließend die Kultur zum Stillstand bringt, um die Synchronisation der Zellen zu ermöglichen.

10. Anwendung des Verfahrens nach einem beliebigen der Ansprüche 1 bis 5 auf die Herstellung von beim Menschen verwendbaren Pfropfteilen, insbesondere Autopfropfteilen, beispielsweise mit dem Ziel der Behandlung von Verbrennungen.

11. Anwendung nach Anspruch 10 integriert mit einer Pfropfungstechnik, die darin besteht, kleine Hautfragmente mit dem Ziel späterer Autopfropfung zu entnehmen, das Pfropfungsstück mit enzymatischen Mitteln 1 bis 2 Wochen vor der Kultur zu fraktionieren, die kultivierten Epidermiszellen auf einem Träger, der aus dünnen Lappen menschlicher Haut oder Schweinehaut besteht, zu vermehren, wobei der Extrakt RE in einer Menge, die mit dem Ziel einer beschleunigten Kultur der Epidermiszellen wirksam ist, zugesetzt wird, und die Pfropfung in klassischer Weise durchzuführen.

12. Pfropfung, die in der Anwendung nach einem der Ansprüche 10 oder 11 durchgeführt oder erhalten wird.

13. Kosmetische, pharmazeutische oder diagnostische Zusammensetzung, enthaltend als Wirkstoff den Extrakt RE und dargeboten mit dem Ziel der Anwendung auf lokalem Wege auf den zu behandelnden Körperteil.

14. Anwendung des Verfahrens nach einem beliebigen der Ansprüche 1 bis 5 auf die Kultur von langer Dauer von Epidermiszellen.

15. Anwendung nach Anspruch 14, wobei man Zellen auf einem Substrat, beispielsweise Kunststoff, in Gegenwart einer wirksamen Menge des Extrakts RE und ohne Zugabe anderer Zellen direkt kultiviert.

Ansprüche (für den Vertragsstaat AT)

1. Verfahren zum Anregen des Wachstums in vitro von Epidermiszellen, insbesondere ausgewachsenen, dadurch gekennzeichnet, daß man die Zellen der Epidermis mit einem wäßrigen Salzextrakt (RE) von Augengewebe in Berührung bringt.

2. Verfahren nach Anspruch 1, dadurch gekennzeichnet, daß das aktive Mittel von Geweben des Auges wie beispielsweise der Gefäßhaut des Auges, der Regenbogenhaut und der Glaskörperflüssigkeit, extrahiert wird.

3. Verfahren nach einem der Ansprüche 1 oder 2, dadurch gekennzeichnet, daß der Extrakt RE durch Behandlung von Augengeweben von Rindern mit einer bei einem pH-Wert nahe 7,2 gepufferten wäßrigen Salzlösung erhalten wird.

4. Verfahren nach irgendeinem der Ansprüche 1 bis 3, dadurch gekennzeichnet, daß man 50 bis 400 µg, als Gesamtprotein, Extrakt pro ml Epidermiszellkultur verwendet, wobei Dosen von 50 bis 100 µg bevorzugt werden.

5. Verfahren nach irgendeinem der Ansprüche 1 bis 4, dadurch gekennzeichnet, daß man menschliche Epidermiszellen kultiviert.

6. Verfahren nach irgendeinem der Ansprüche 1 bis 5, dadurch gekennzeichnet, daß man die Chromosomen der menschlichen Epidermiszelle insbesondere zur Entdeckung von natürlichen oder induzierten Anomalien der Chromosomen untersucht.

7. Verfahren nach Anspruch 6, dadurch gekennzeichnet, daß man die Austausche von Schwesterchromatiden (SCE) in Kulturen in vitro von menschlichen ausgewachsenen Keratinozyten bewertet.

8. Verfahren nach irgendeinem der Ansprüche 1 bis 7, dadurch gekennzeichnet, daß man Epidermiszellen in Abwesenheit des Wachstumsfaktors RE in eine vorherige normale Kultur bringt, daß man nach einer Zeit zwischen 5 und 10 Tagen, beispielsweise nach 8 Tagen, in die Kultur eine wirksame RE-Menge einführt und daß man einen oder zwei Tage danach eine weitere wirksame RE-Menge einführt, um eine zweite Replikation der Zellen zu erhalten.

9. Verfahren nach irgendeinem der Ansprüche 1 bis 7, dadurch gekennzeichnet, daß man Epidermiszellen in Kultur bringt, indem man anfangs eine wirksame RE-Menge einführt und anschließend die Kultur zum Stillstand bringt, um die Synchronisation der Zellen zu ermöglichen.

10. Verfahren nach irgendeinem der Ansprüche 1 bis 9, dadurch gekennzeichnet, daß man Pfropfteile und insbesondere beim Menschen mit dem Ziel der Behandlung von Verbrennungen verwendbare Autopfropfteile herstellt.

11. Verfahren nach Anspruch 10, dadurch gekennzeichnet, daß man es mit einer Pfropfungstechnik integriert, die darin besteht, kleine Hautfragmente mit dem Ziel späterer Autopfropfung zu entnehmen, das Pfropfungsstück mit enzymatischen Mitteln 1 bis 2 Wochen vor der Kultur zu fraktionieren, die kultivierten Epidermiszellen auf einem Träger, der aus dünnen Lappen menschlicher Haut oder Schweinehaut besteht, zu vermehren, wobei der Extrakt RE in einer Menge, die mit dem Ziel einer beschleunigten Kultur der Epidermiszellen wirksam ist, zugesetzt wird, und die Pfropfung in klassischer Weise durchzuführen.

12. Verfahren nach irgendeinem der Ansprüche 1 bis 11, dadurch gekennzeichnet, daß man eine Kultur von langer Dauer von Epidermiszellen durchführt.

13. Verfahren nach irgendeinem der Ansprüche 1 bis 14, dadurch gekennzeichnet, daß man die Zellen auf einem Substrat, beispielsweise Kunststoff, in Gegenwart einer wirksamen Menge des Extrakts RE und ohne Zugabe anderer Zellen direkt kultiviert.

14. Verfahren zur Herstellung einer kosmetischen, pharmazeutischen oder diagnostischen Zusammensetzung, dadurch gekennzeichnet, daß man den nach dem Verfahren nach einem der Ansprüche 1 bis 13 erhaltenen Extrakt RE mit einem Träger mischt, der für den Auftrag auf lokalem Wege auf den zu behandelnden Körperteil.

0 032 145